# EUROPEAN PATENT SPECIFICATION

(11) **EP 3 128 310 B1**
(45) Date of publication and mention of the grant of the patent: **20.10.2021**
(21) Application number: 15772509.4
(22) Date of filing: 12.03.2015
(51) Int. Cl.: G01N 21/55, G01N 15/14, G01N 33/543, G01N 15/00, G01N 15/06

(54) **ANALYSIS DEVICE AND ANALYSIS METHOD**
ANALYSEVORRICHTUNG UND ANALYSEVERFAHREN
DISPOSITIF D'ANALYSE ET PROCÉDÉ D'ANALYSE

(30) Priority: 31.03.2014 JP 2014072552
(43) Date of publication of application: 08.02.2017
(73) Proprietor: JVC Kenwood Corporation, Yokohama-shi, Kanagawa 221-0022 (JP)
(72) Inventor: ONO, Masayuki, Yokohama-shi, Kanagawa 221-0022 (JP); YAGYU, Shingo, Yokohama-shi, Kanagawa 221-0022 (JP); ITONAGA, Makoto, Yokohama-shi, Kanagawa 221-0022 (JP); HASEGAWA, Yuichi, Yokohama-shi, Kanagawa 221-0022 (JP); TSUJITA, Koji, Yokohama-shi, Kanagawa 221-0022 (JP)
(74) Representative: Klang, Alexander H.
(86) International application number: PCT/JP2015/057304
(87) International publication number: WO 2015/151753

(56) References cited:
- JP-A- 2004 309 288
- JP-A- 2005 156 538
- JP-A- 2012 237 711
- JP-A- 2013 064 722
- US-A1- 2008 068 952
- US-A1- 2012 288 408
- US-B1- 6 829 208
- KOJI TSUJITA ET AL: "Ultrahigh-Sensitivity Biomarker Sensing System Based on the Combination of Optical Disc Technologies and Nanobead Technologies", JAPANESE JOURNAL OF APPLIED PHYSICS, vol. 52, no. 9S2, 20 September 2013 (2013-09-20), page 09LB02, XP055228014, JP ISSN: 0021-4922, DOI: 10.7567/JJAP.52.09LB02
- KOJI TSUJITA ET AL.: 'Ultrahigh-Sensitivity Biomarker Sensing System Based on the Combination of Optical Disc Technologies and Nanobead Technologies' JAPANESE JOURNAL OF APPLIED PHYSICS vol. 52, 2013, pages 09LB02 - 1- 09LB02-4, XP055228014
- MASAYUKI ONO ET AL.: 'Hikari Disc Gijutsu o Oyo shita Biomaker Koseido Kenshutsu Gijutsu no Kaihatsu' SOCIETY FOR CHEMISTRY AND MICRO-NANO SYSTEMS DAI 27 KAI KENKYUKAI KOEN YOSHISHU vol. 27 TH, May 2013, page 23, XP008183992

## Description

### TECHNICAL FIELD

The present invention relates to an analysis device and an analysis method for analyzing biomaterials such as antibodies and antigens.

### BACKGROUND ART

Immunoassays are known that quantitatively analyze disease detection and therapeutic effects by detecting particular antigens or antibodies as bi omarkers associated with diseases. One of the immunoassays is an enzyme-linked immunosorbent assay (ELISA) for detecting antigens or antibodies labeled by enzymes, which is widely used because of having the advantage of low costs. The ELISA requires a long period of time, such as from several hours to a day, to complete a series of multiple steps including pretreatment, antigen-antibody reaction, bond/free (B/F) separation, and enzyme reaction.

Another technology is disclosed in which antibodies fixed to an optical disc are allowed to bind to antigens in a specimen, and the antigens are further bound to particles having antibodies and then scanned with an optical head, so as to count the particles captured on the disc in a short period of time (JP H05-005741 A). Still another technology is disclosed in which biosamples or particles are adsorbed to a surface of an optical disc on which a tracking structure is formed, so as to detect changes in signal by an optical pickup (JP 2002-530786 A) .

KOJI TSUJITA ET AL, "Ultrahigh-SensitiviLy Biomarker Sensing System Based on the Combination of Optical Disc Technologies and Nanobead Technologies", JAPANESE JOURNAL OF APPLIED PHYSICS, discloses an ultrahigh-sensitivity biomarker sensing system based on the combination of optical disc technologies and nanobead technologies.

US 2012/288408 A discloses a sample analysis disc which has concave sections and convex sections formed alternately in a track area of a disc surface. Labeled beads are immobilized to the track area. Each labeled bead has a biopolymer bound thereto. Only one of the labeled beads is allowed to be filled in each concave section.

### SUMMARY OF INVENTION

The technology disclosed in JP H05-005741 A and JP 2002-530786 A, however, may fail to obtain detection signals corresponding to particles depending on the type and arrangement of the particles used. Such failure leads to inaccurate counting results, which may decrease the performance of quantitative analysis of analytes.

In view of the foregoing, an object of the present invention is to provide an analysis device and an analysis method with improved quantitative analysis of analytes.

In accordance with the present invention, an analysis device and a method set defined in the appended claims is provided. Further embodiments are inter alia disclosed in the dependent claims. In particular, in order to achieve the object, a first aspect of the present invention is an analysis device including: an optical scanning unit configured to optically scan a surface of a substrate to which analytes and particles for labeling the analytes are fixed; a pulse detector configured to detect a pulse wave and a pulse width of the pulse wave included in a detection signal obtained from the optical scanning unit when the optical scanning unit scans the substrate; and a counting unit configured to count the analytes and determine that an analyte count is one when the pulse detector consecutively detects two pulse waves each having a pulse width less than a first reference value.

A second aspect of the present invention is an analysis method including: optically scanning a surface of a substrate to which analytes and particles for labeling the analytes are fixed; detecting a pulse wave and a pulse width of the pulse wave included in a detection signal obtained by scanning the substrate; and counting the analytes and determining that an analyte count is one when consecutively detecting two pulse waves each having a pulse width less than a first reference value in the detection signal.

### BRIEF DESCRIPTION OF DRAWINGS

[Fig. 1] Fig. 1 is a schematic block diagram for describing a fundamental configuration of an analysis device according to an embodiment of the present invention.
[Fig. 2] Fig. 2(a) to Fig. 2(f) are enlarged cross-sectional views each schematically showing a substrate of the analysis device according to the embodiment of the present invention, for describing an example of a method of fixing antibodies, antigens, and beads to the substrate.
[Fig. 3] Fig. 3 is a schematic view for describing a state in which two beads 66 are bound to one exosome on the substrate.
[Fig. 4] Fig. 4 is a view showing simulation results of detection of adjacent beads while varying the number of beads, for describing characteristics of a spot position and signal intensity when scanning the substrate.
[Fig. 5] Fig. 5 is a view for describing characteristics between a spot position and signal intensity when scanning the substrate of the analysis device according to the embodiment of the present invention, in which adjacent beads are detected while varying the number of beads.
[Fig. 6] Fig. 6 is a view for describing a method of determining a reference value stored in a storage unit included in the analysis device according to the embodiment of the present invention.
[Fig. 7] Fig. 7 is a view for describing a method of determining a reference value stored in the storage unit included in the analysis device according to the embodiment of the present invention.
[Fig. 8] Fig. 8 is a flowchart for describing the operation of the analysis device according to the embodiment of the present invention.
[Fig. 9] Fig. 9 is a view for describing the operation of the analysis device according to the embodiment of the present invention.
[Fig. 10] Fig. 10 is a view for describing the operation of the analysis device according to the embodiment of the present invention.
[Fig. 11] Fig. 11 is a view for comparing characteristics of biomarker concentration and counting results of beads in the analysis device according to the embodiment of the present invention with those in a conventional device.

### DESCRIPTION OF EMBODIMENTS

Hereinafter, an embodiment of the present invention will be described with reference to the drawings. The same or similar elements shown in the drawings are designated by the same or similar reference numerals below, and overlapping descriptions thereof are not repeated herein.

### [Analysis Device]

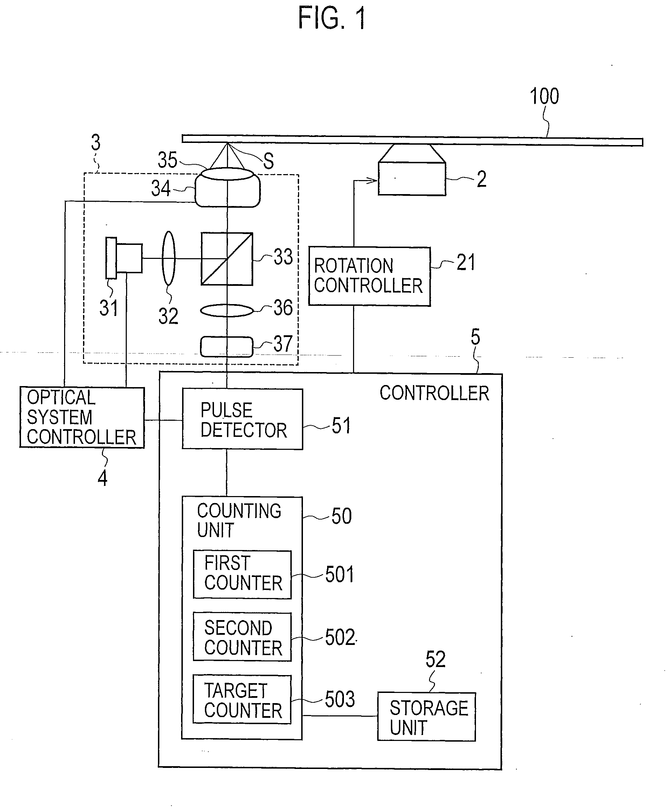

As shown in Fig. 1, an analysis device according to the embodiment of the present invention includes a substrate 100, a motor 2 that rotates the substrate 100, an optical scanning unit 3 that optically scans the substrate 100, and a controller 5 that controls the motor 2 and the optical scanning unit 3.

The substrate 100 is formed into a circular shape having substantially the same dimensions as optical discs such as compact discs (CDs), digital versatile discs (DVDs), and Blu-ray discs (BD). The substrate 100 has a track structure on the surface thereof that the optical scanning unit 3 can scan. The track structure includes, for example, grooves, lands, and pits, and is formed into a spiral extending from the inner side to the outer side. The substrate 100 is formed of a hydrophobic resin material, such as polycarbonate resin and cycloolefin polymer, used for common optical discs. The substrate 100 may be, as necessary, provided with a thin film on the surface thereof, or subjected to surface treatment with a silane coupling agent.

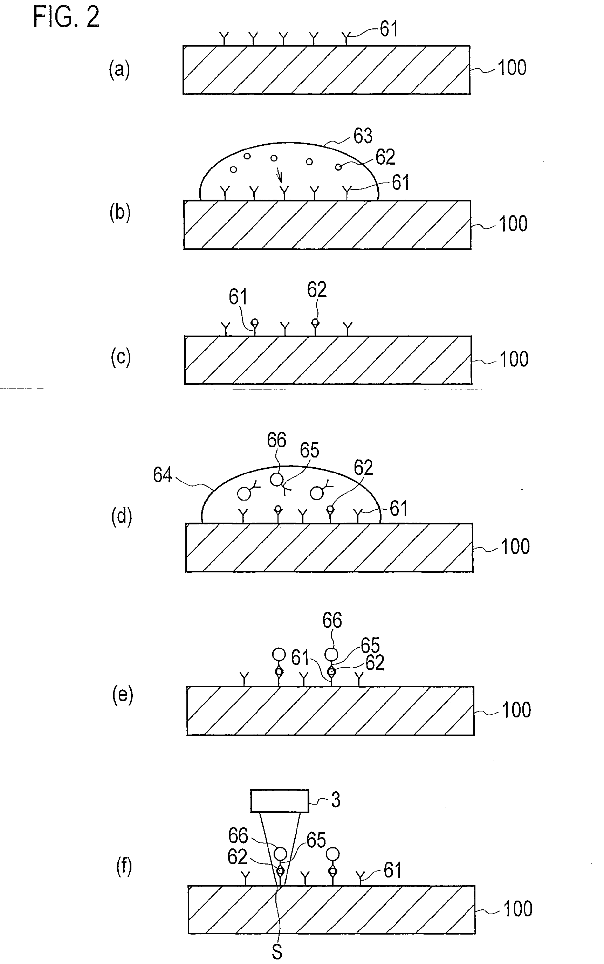

As shown in Fig. 2, antibodies 61 specifically binding to antigens 62, which are biomaterials serving as analytes, are fixed to the surface of the substrate 100. The antigens 62 are labeled with beads (particles) 66 to which antibodies 65 specifically binding to the antigens 62 are adsorbed, so that the antigens 62 and the beads 66 are correlatively fixed to the surface of the substrate 100. The antigens 62 are specifically bound to the antibodies 61 and 65, so as to be used as biomarkers serving as indicators of diseases.

As shown in Fig. 2 (a), the antibodies 61 are preliminarily fixed to the surface of the substrate 100. The antibodies 61 are bound to the surface of the substrate 100 due to hydrophobic binding or covalent binding. The antibodies 61 may be fixed to the surface of the substrate 100 via a substance such as avidin. Then, as shown in Fig. 2 (b), a sample solution 63 including the antigens 62 is applied dropwise to the surface of the substrate 100. The antigens 62 move through the sample solution 63 by Brownian motion and come into contact with the antibodies 61, so as to be specifically bound to the antibodies 61 by an antigen-antibody reaction. As shown in Fig. 2(c), the surface of the substrate 100 to which the sample solution 63 is applied dropwise is subjected to spin washing with pure water or the like, so as to remove the sample solution 63 including excessive antigens 62 not bound to the antibodies 61 from the surface of the substrate 100.

As shown in Fig. 2(d), a buffer solution 64 including the beads 66 is applied dropwise to the surface of the substrate 100. The buffer solution 64 may be applied while the sample solution 63 remains on the surface of the substrate 100. The antibodies 65 adsorbed to the beads 66 specifically bind to the antigens 62 by the antigen-antibody reaction. The beads 66 are then bound to the antigens 62, so as to label the antigens 62.

The beads 66 are formed of synthetic resin such as polystyrene including a magnetic material such as ferrite, and formed into a substantially spherical shape. A diameter of the beads 66 is in the range of from several tens of nanometers to several hundreds of nanometers, and a particular example of the diameter is 200 nm. When the buffer solution 64 is applied dropwise, the beads 66 are quickly collected to the surface of the substrate 100 such that a magnet is placed on the opposite side of the surface of the substrate 100, so as to promote the reaction with the antigens 62. In addition, the time required to label the antigens 62 fixed to the substrate 100 can be reduced to approximately several minutes such that the antigens 62 and the beads 66 are simultaneously applied to the substrate 100.

The antibodies 61 and 65 may be any biomaterials having specificity that specifically bind to the antigens 62. A combination of the antibodies 61 and 65 is selected such that the antibodies 61 and 65 separately bind to different sites. For example, when membrane vesicles such as exosomes on which several types of antigens 62 are expressed are used as analytes, the types of the antibodies 61 and 65 are chosen differently from each other, so as to detect a biosample including two types of antigens 62. The antibodies 61 and 65 are, however, not limited thereto, and may be the same type because exosomes, which are different from typical antigens, include multiple antigens of the same kind of protein on the surface thereof.

As shown in Fig. 2(e), the substrate 100 to which the buffer solution 64 is applied dropwise is washed with, for example, pure water, so as to remove the buffer solution 64 including excessive beads 66 not bound to the antigens 62 from the substrate 100. As shown in Fig. 2(f), the substrate 100 is optically scanned by the optical scanning unit 3, so as to detect the beads 66 to analyze the antigens 62 labeled with the beads 66.

As shown in Fig. 1, the optical scanning unit 3 includes a laser oscillator 31, a collimator lens 32, a beam splitter 33, an actuator 34, an objective lens 35, a condensing lens 36, and a light detector 37. The optical scanning unit 3 is an optical pickup that optically scans the substrate 100.

The laser oscillator 31 emits laser light to the collimator lens 32 according to the control by the controller 5. The laser oscillator 31 is a semiconductor laser oscillator that emits laser light having, for example, a wavelength of 405 nm which is the same as that for reproduction of BD, and output of about 1 mW. The collimator lens 32 collimates the laser light emitted from the laser oscillator 31. The beam splitter 33 reflects the laser light collimated by the collimator lens 32 toward the objective lens 35.

The objective lens 35 concentrates the laser light transmitted via the beam splitter 33 on the surface of the substrate 100, to which the antibodies 61 are fixed, due to the operation of the actuator 34 according to the control by the controller 5, so as to image spot S. The objective lens 35 has a numerical aperture of, for example, 0.85. The laser light concentrated by the objective lens 35 is reflected from the substrate 100 and then reaches the beam splitter 33. The incident laser light passes through the beam splitter 33 and further reaches the light detector 37 via the condensing lens 36. The condensing lens 36 concentrates the laser light reflected from the substrate 100 into the light detector 37. The light detector 37 is, for example, a photodiode to output, to the controller 5, a detection signal corresponding to the volume of the laser light reflected from the substrate 100.

The controller 5 controls the operation of the motor 2 via a rotation controller 21. The motor 2 is controlled by the controller 5 to rotate the substrate 100 at a constant linear velocity (CLV) . The linear velocity is, for example, 4.92 m/s.

The controller 5 controls the operation of the laser oscillator 31 and the actuator 34 via an optical system controller 4. The actuator 34 is controlled by the controller 5 to move the optical scanning unit 3 in a radial direction of the substrate 100 so as to spirally scan the surface of the rotating substrate 100. The controller 5 also detects errors such as focus errors (FE) or tracking errors (TE) from the detection signal output from the light detector 37. The controller 5 controls the actuator 34 and other components to appropriately scan the surface of the substrate 100 depending on the errors detected.

The controller 5 includes a pulse detector 51, a storage unit 52, and a counting unit 50. The pulse detector 51 inputs the detection signal output from the light detector 37. The pulse detector 51 detects a pulse wave and a pulse width of the pulse wave included in the detection signal obtained from the optical scanning unit 3. The pulse detector 51 is a signal processing device such as a digital signal processor (DSP) . The storage unit 52 is a memory such as a semiconductor memory. The storage unit 52 stores reference values corresponding to the pulse wave and the pulse width detected by the pulse detector 51.

The counting unit 50 counts the number of analytes fixed to the surface of the substrate 100 according to the pulse wave detected by the pulse detector 51 and the reference values stored in the storage unit 52. The counting unit 50 is, for example, a central processing unit (CPU) . The counting unit 50 includes, as a logical structure, a first counter 501, a second counter 502, and a target counter 503.

The first counter 501 measures pulse width Ta of the pulse wave detected by the pulse detector 51. The second counter 502 measures, depending on the pulse width Ta of the pulse wave detected by the pulse detector 51, pulse interval Tb between the pulse wave and a pulse wave subsequently detected. The target counter 503 counts the number of beads 66 so as to count the analytes according to the measurement results by the first counter 501 and the second counter 502 and the reference values stored in the storage unit 52.

In the example of Fig. 2(d), the beads 66 are assumed to label biomaterials such as exosomes to which a plurality of antigens 62 are adsorbed. Typically, beads 66 are excessively applied to a solution as compared with the amount of exosomes on the premise that a minute amount of exosomes is used for analysis, such as for detection of cancer in early stages. Exosomes typically have various diameters in the range of from about 50 nm to about 150 nm, while beads 66 have a diameter of approximately 200 nm.

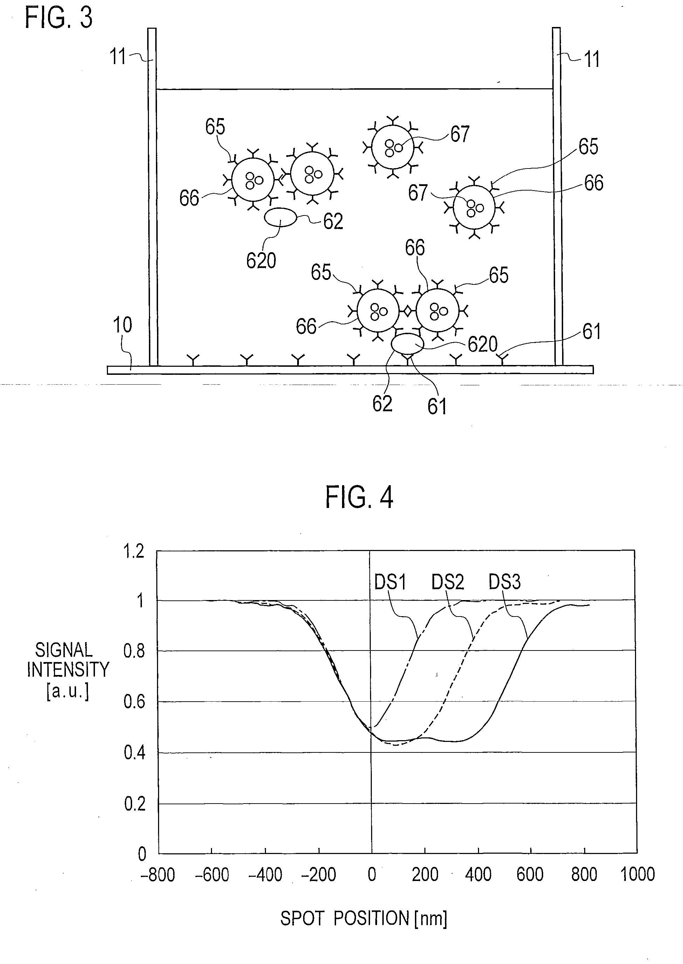

For example, as shown in Fig. 3, two beads 66 are often bound to one exosome 620 in which one of antigens 62 on the surface thereof is bound to an antibody 61 on the substrate 100. In other words, when detecting exosomes 620 by optically scanning beads 66, the probability is extremely high that two pulse waves corresponding to two beads 66 adjacent to each other denote the presence of one exosome 620 having two antigens 62 labeled by antibodies 65 of the respective two beads 66. Fig. 3 shows an example in which a well 11 having a penetration hole is provided on the upper surface of the substrate 100, so that the penetration hole of the well 11 and the substrate 100 form a container to which liquid is applied dropwise.

The analysis device according to the embodiment of the present invention determines whether the pulse wave detected is derived-from two beads 66 adjacent to each other according to the detection signal and the reference values stored in the storage unit 52. When a pattern of the pulse wave determined to be derived from adjacent two beads 66 is detected, the analysis device counts exosomes 620 present within a corresponding scanning region and determines that the exosome count is one, so as to improve quantitative analysis of the exosomes 620 as analytes.

### -Reference Values-

Fig. 4 shows simulation results of three detection signals DS1 to DS3 obtained in such a manner as to scan each of one projection pit assuming that one bead is present on the substrate 100, adjacent two projection pits assuming that two beads are present on the substrate 100, and adjacent three projection pits assuming that three beads are present on the substrate 100. The transverse axis represents a position of the spot S corresponding to each front bead 66 in a particular interval, and the vertical axis represents signal intensity obtained such that each signal is normalized by a detection signal detected when there is no bead 66. The pulse width is assumed to gradually increase as the number of beads 66 increases, as indicated by the detection signal DS1 with one isolated bead 66, the detection signal DS2 with two beads 66, and the detection signal DS3 with three beads 66 in this order.

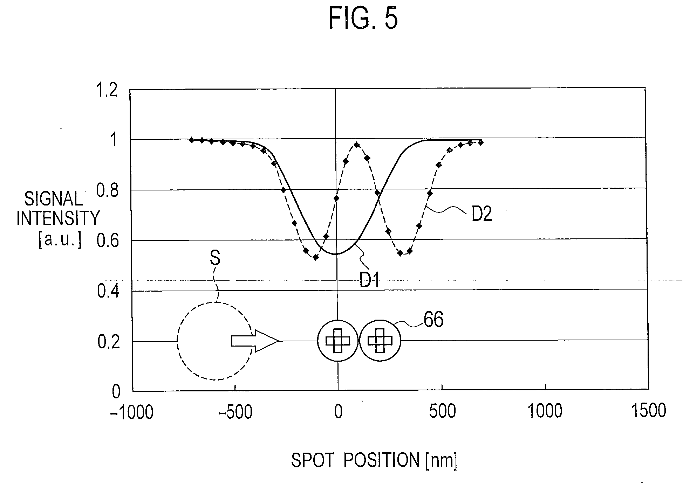

As shown in Fig. 5, however, a detection signal D2 obtained such that adjacent two beads 66 are actually scanned includes two pulse waves with substantially the same pulse width which is smaller than a pulse width of a detection signal D1 obtained such that one isolated bead 66 is scanned. A detection signal with adjacent three beads 66 scanned also includes two pulse waves each having substantially the same width as those of the detection signal D2 and having a larger pulse interval than the detection signal D2.

When the diameter of beads 66 is approximately one half of the wavelength of the laser light scanned, and there are a plurality of beads 66 adjacent to each other, the number of beads 66 cannot be counted accurately, which may decrease the performance of quantitative analysis of the analytes. The inventors resolved the effects of light on a structure (particles) with a smaller size than a wavelength of the light having different pits from common optical discs as described above, by solving Maxwell's equations with regard to times and space variables by a finite-difference time-domain (FDTD) method.

When two adjacent beads 66 are present on the substrate 100, the probability is high that the two beads 66 label one exosome 620. The analysis device according to the embodiment of the present invention can count the number of exosomes 620 with high accuracy on the basis of the pulse width Ta and the pulse interval Tb of the detection signal depending on the arrangement of beads 66 and the reference values stored in the storage unit 52, as described below.

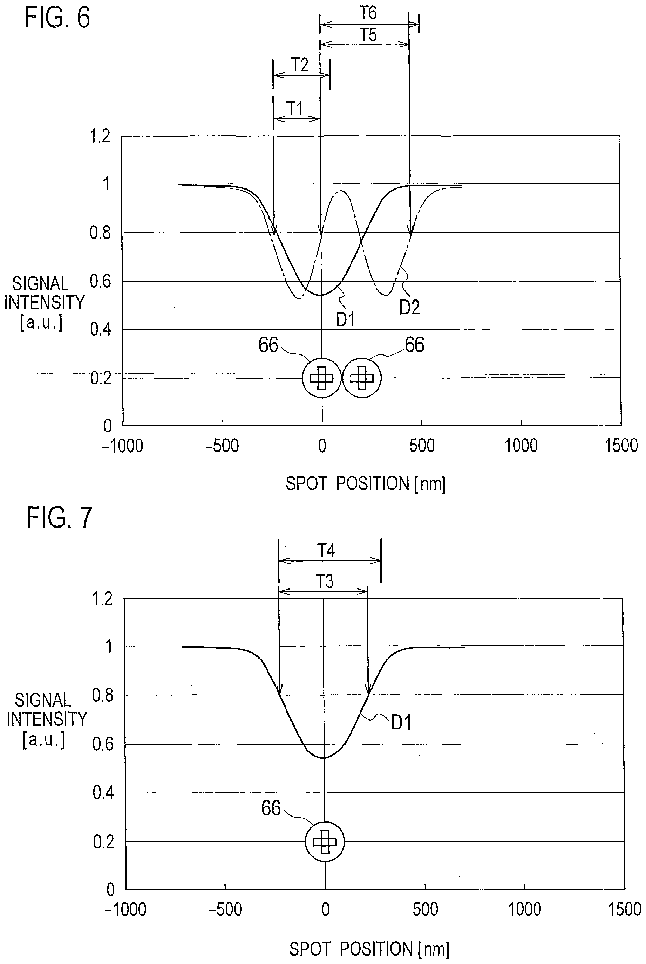

As shown in Fig. 6, the storage unit 52 preliminarily stores first pulse width T1 of the detection signal D2 having two pulse waves and first reference value T2 determined depending on the first pulse width T1 when the optical scanning unit 3 scans two beads 66 adjacent to each other. The first reference value T2 is, for example, the sum of the first pulse width T1 and a predetermined value. The predetermined value added to the first pulse width T1 may be a jitter value included in the detection signal. The predetermined value added to the first pulse width T1 may also be approximately 100% to 130% of the jitter value. The first reference value T2 may be a predetermined percentage of the first pulse width T1. For example, the first reference value T2 is approximately 100% to 130% of the first pulse width T1.

As shown in Fig. 7, the storage unit 52 preliminarily stores second pulse width T3 of the detection signal D1 and second reference value T4 determined depending on the second pulse width T3 when the optical scanning unit 3 scans a bead 66 isolated from other beads 66. The second reference value T4 is, for example, the sum of the second pulse width T3 and a predetermined value. The predetermined value added to the second pulse width T3 may be a jitter value included in the detection signal. The predetermined value added to the second pulse width T3 may also be approximately 100% to 130% of the jitter value. The second reference value T4 may be a predetermined percentage of the second pulse width T3. For example, the second reference value T4 is approximately 100% to 130% of the second pulse width T3.

Further, as shown in Fig. 6, the storage unit 52 preliminarily stores pulse interval T5 of two pulse waves included in the detection signal D2 and third reference value T6 determined depending on the pulse interval T5 when the optical scanning unit 3 scans the two adjacent beads 66. The third reference value T6 is, for example, the sum of the pulse interval T5 and a predetermined value. The predetermined value added to the pulse interval T5 may be a jitter value included in the detection signal. The predetermined value added to the pulse interval T5 may also be approximately 100% to 130% of the jitter value. The third reference value T6 may be a predetermined percentage of the pulse width T5. For example, the third reference value T6 is approximately 100% to 130% of the pulse width T5.

### <Analysis Method>

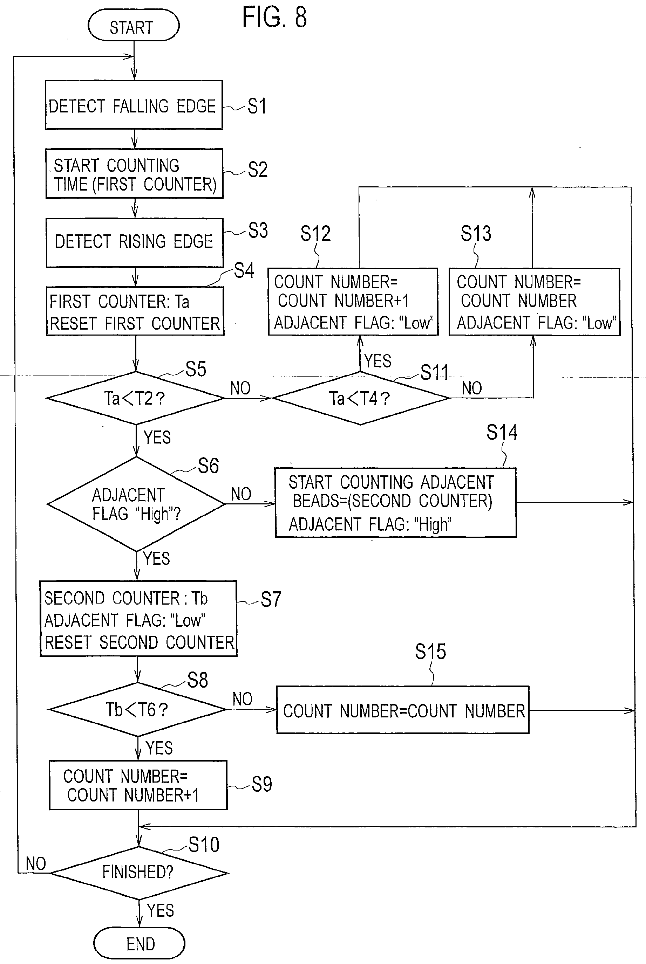

An analysis method by the analysis device according to the embodiment of the present invention is described below with reference to the flowchart shown in Fig. 8, in which the optical scanning unit 3 optically scans the substrate 100, and the counting unit 50 counts the number of exosomes 620 as analytes fixed to the substrate 100.

First, the operator allows the rotation controller 21 and the optical system controller 4 to respectively start operations of the motor 2 and the optical scanning unit 3 according to the control by the controller 5. The substrate 100 to which antigens 62 and beads 66 are fixed on the surface thereof by the antigen-antibody reaction, is rotated at a constant linear velocity by the motor 2, so as to be optically scanned by the optical scanning unit 3. The optical scanning unit 3 detects, with the light detector 37, the laser light emitted from the laser oscillator 31 and reflected from the surface of the substrate 100. The light detector 37 outputs a detection signal corresponding to the volume of the detected laser light to the pulse detector 51.

In step S1, the pulse detector 51 obtains the detection signal output from the light detector 37 to detect a falling edge of the obtained detection signal. The pulse detector 51 preliminarily holds a threshold set to intensity corresponding to approximately one half of a peak value of the detection signal detected when beads 66 are scanned, and detects a point where the detection signal falls below the threshold as a falling edge of the detection signal.

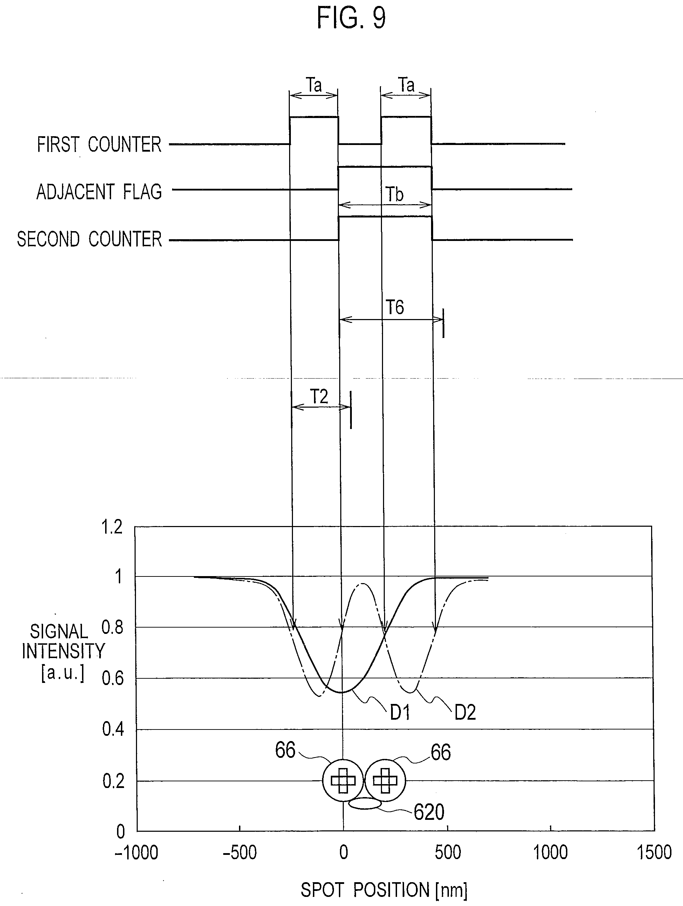

In step S2, the first counter 501 starts measuring time Ta from the point where the falling edge is detected in step S1, as shown in Fig. 9.

In step S3, the pulse detector 51 detects a rising edge of the detection signal obtained from the light detector 37. The pulse detector 51 preliminarily holds the threshold set to the intensity corresponding to approximately one half of the peak value of the detection signal detected when beads 66 are scanned, and detects a point where the detection signal exceeds the threshold as a rising edge of the detection signal.

In step S4, the first counter 501 fixes the time Ta from the point where the falling edge is detected in step S1 to the point where the rising edge is detected in step S3, and resets it. The target counter 503 obtains and holds the time Ta fixed by the first counter 501 as a pulse width (half width) Ta of the pulse wave detected in steps S1 to S3.

In step S5, the target counter 503 reads out the first reference value T2 from the storage unit 52, and determines whether the pulse width Ta held in step S4 is less than the first reference value T2. The target counter 503 sets the process proceeding to step S6 when the pulse width Ta is less than the first reference value T2, or sets the process proceeding to step S11 when the pulse width Ta is greater than or equal to the first reference value T2.

When the pulse width Ta is less than the first reference value T2 in step S5, the target counter 503 determines whether an adjacent flag is "High" (=1) in step S6. The adjacent flag is a flag set in the target counter 503 in association with the second counter 502. The target counter 503 sets the process proceeding to step S7 when the adjacent flag is "High" in step S6, or sets the process proceeding to step S14 when the adjacent flag is "Low" (=0).

In the example shown in Fig. 9, it is assumed that the detection signal D2 is input into the pulse detector 51, and the pulse detector 51 detects the rising edge of the first pulse wave in step S3. In such a case, since the adjacent flag is "Low" in step S6, the counting unit 50 sets the process proceeding to step S14.

In step S14, the second counter 502 starts measuring time Tb from the point where the rising edge is detected in step S3. The target counter 503 sets, in association with the second counter 502, the adjacent flag to "High" from the point where the rising edge is detected in step S3, and the process proceeds to step S10.

In step S10, the controller 5 determines whether the scanning of the substrate 100 in a predetermined tracking region by the optical scanning unit 3 is finished. The controller 5 ends the process when the scanning is finished, or sets the process returning to step S1 when the scanning is not yet finished.

In the example shown in Fig. 9, it is assumed that the detection signal D2 is input into the pulse detector 51, and the pulse detector 51 detects the rising edge of the second pulse wave in step S3. In such a case, since the adjacent flag is "High" in step S6, the counting unit 50 sets the process proceeding to step S7.

In step S7, the second counter 502 fixes the time Tb from the point where the first rising edge is detected in step S3 to the point where the second rising edge is detected in the next step S3, and resets it. The target counter 503 obtains and holds the time Tb fixed by the second counter 502 as a pulse interval Tb of the two pulse waves detected in the two sets of steps S1 to S3, and sets the adjacent flag to "Low".

In step S8, the target counter 503 reads out the third reference value T6 from the storage unit 52, and determines whether the pulse interval Tb held in step S7 is less than the third reference value T6. The target counter 503 sets the process proceeding to step S9 when the pulse interval Tb is less than the third reference value T6, or sets the process proceeding to step S15 when the pulse interval Tb is greater than or equal to the third reference value T6.

When the pulse interval Tb is less than the third reference value T6 in step S8, the target counter 503 determines in step S9 that the optical scanning unit 3 has scanned the two adjacent beads 66 and therefore the count of exosomes 620 results in one, so as to set the process proceeding to step S10. In the example shown in Fig. 9, when the detection signal D2 is input into the pulse detector 51, the number of beads 66 is two. Since the probability is extremely high that the two adjacent beads 66 are bound to one exosome 620, the count of exosomes 620 results in one. Thus, the target counter 503 determines that the number of exosomes 620 counted is one when consecutively detecting the two pulse waves between which the pulse interval Tb is less than the third reference value T6.

When the pulse interval Tb is greater than or equal to the third reference value T6 in step S9, the target counter 503 determines in step S15 that the pulse waves having the pulse width greater than or equal to the third reference value T6 are noise derived from foreign substances or aggregations, so as not to consider the pulse waves when implementing counting processing. In other words, three or more beads 66 adjacent to each other are required to be bound to a plurality of exosomes 620 on the substrate 100, and the probability of expression is quite low. Thus, the pulse waves having the pulse width greater than or equal to the third reference value T6 are ignored as being noise when implementing counting processing.

When the pulse width Ta is greater than or equal to the first reference value T2 in step S5, the target counter 503 reads out the second reference value T4 from the storage unit 52, and determines in step S11 whether the pulse width Ta held in step S4 is less than the second reference value T4. The target counter 503 sets the process proceeding to step S12 when the pulse width Ta is less than the second reference value T4, or sets the process proceeding to step S13 when the pulse width Ta is greater than or equal to the second reference value T4.

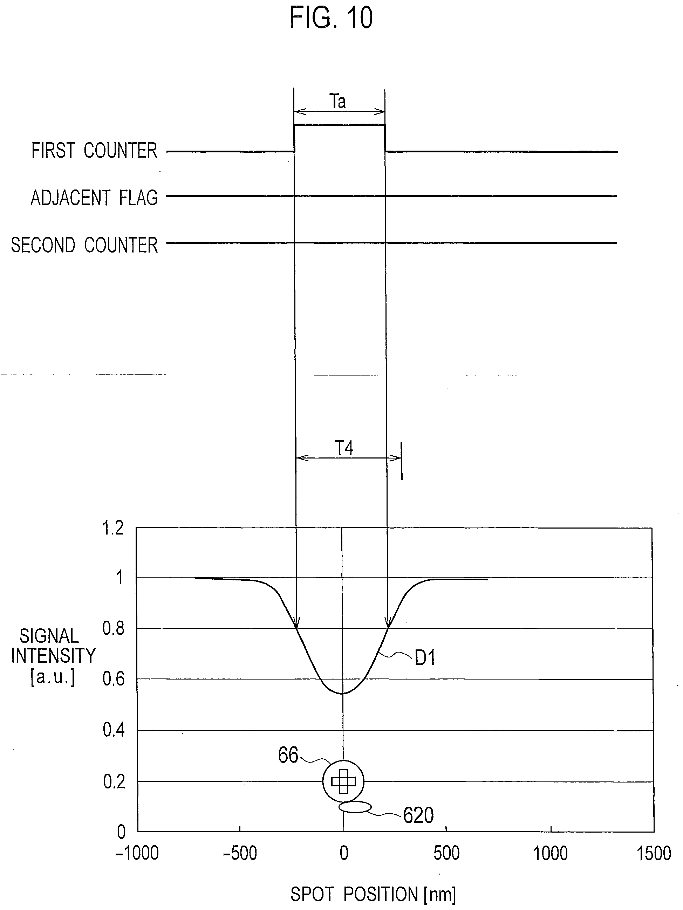

As shown in the example of Fig. 10, it is assumed that the detection signal D1 is input into the pulse detector 51, and the pulse detector 51 detects the rising edge of the pulse wave in step S3. In this case, the pulse width Ta is greater than or equal to the first reference value T2 in step S5, and the pulse width Ta is less than the second reference value T4 in step S9, so that the counting unit 50 sets the process proceeding to step S12.

In step S12, the target counter 503 determines that the optical scanning unit 3 has scanned one bead 66 isolated from other beads 66 and therefore the count of beads 66 results in one. The probability is high that the isolated bead 66 is bound to one exosome 620 on the substrate 100. Thus, the target counter 503 determines that the count of exosomes 620 is one when the pulse wave having the pulse width Ta greater than or equal to the first reference value T2 and less than the second reference value T4 is detected. The target counter 503 then sets the adjacent flag to "Low", and the process proceeds to step S10.

When the pulse width Ta is greater than or equal to the second reference value T4 in step S11, the target counter 503 determines in step S13 that the pulse wave having the pulse width greater than or equal to the second reference value T4 is noise derived from foreign substances or aggregations, so as not to consider the pulse wave when implementing counting processing. The target counter 503 then sets the adjacent flag to "Low", and the process proceeds to step S10.

It is also assumed that the pulse wave having the pulse width Ta less than the first reference value T2 is detected in the first set of steps S1 to S3, and the pulse wave having the pulse width Ta greater than or equal to the first reference value T2 and less than the second reference value T4 is detected in the next set of steps S1 to S3. In such a case, the target counter 503 determines that the pulse wave detected first is noise derived from foreign substances or aggregations, so as not to consider the pulse wave when implementing counting processing.

As described above, when the pulse wave having the pulse width Ta less than the first reference value T2 is consecutively detected twice, the target counter 503 adds 1 to count up the number of exosomes 620. When the pulse wave having the pulse width Ta greater than or equal to the first reference value T2 and less than the second reference value T4 is detected in the detection signal, the target counter 503 adds 1 to count up the number of beads 66.

### <Comparative Example>

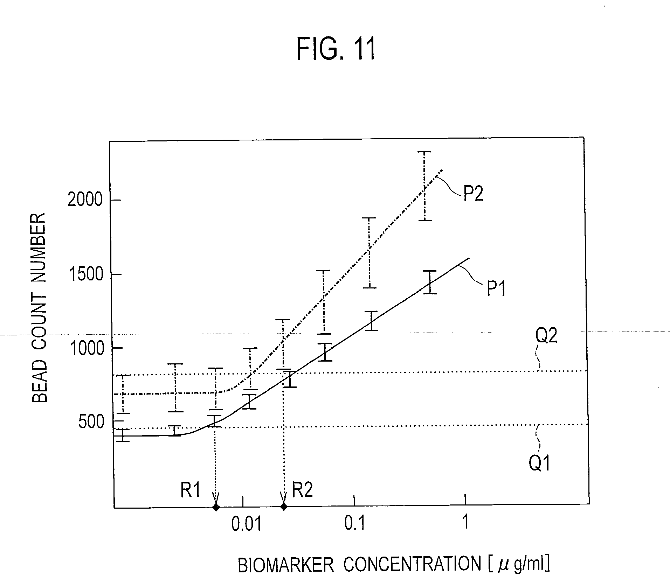

A comparative example in which the counted results of beads 66 obtained by the analysis device according to the embodiment of the present invention are compared with the counted results obtained by a conventional method, is described below with reference to Fig. 11. The transverse axis represents concentration of biomarkers (exosomes 620) as analytes, and the vertical axis represents the counted results of beads 66. The counted results obtained by the analysis device according to the embodiment of the present invention are indicated by the curved line P1, and the counted results obtained by the conventional method are indicated by the curved line P2.

The analysis revealed that the count is entirely smaller in the curved line P1 than the curved line P2 regardless of the biomarker concentration. When the biomarker content is zero, the count would ideally result in zero. In the detection method by use of the antigen-antibody reaction, however, nonspecific adsorption appears on the substrate 100 other than the binding by the antigen-antibody reaction. Even when the biomarker concentration is zero, the beads 66 fixed to the surface of the substrate 100 due to the nonspecific adsorption are thus inevitably counted.

In the respective curved lines P1 and P2, the upper limits of error Q1 and Q2 at which the biomarker concentration of the respective curved lines P1 and P2 is zero (plots at the leftmost end in Fig. 11) are referred to as background noise. The points of contact (points of intersection) between the background noise and the lower limits of error at each plot are respectively denoted by the limits of detection R1 and R2. The counted results by the conventional method each denote the actual number of beads 66 and vary in each test (analysis), and therefore lead to an increase in variation. The counted results by the analysis device according to the embodiment of the present invention are obtained according to "the count of beads = the number of exosomes", so as to minimize variations even when the test (analysis) is repeated. The limit of detection R1 in the analysis device according to the embodiment of the present invention is therefore improved compared with the limit of detection R2 in the conventional method, and it is apparent that the sensitivity of the biomarker detection is improved. Accordingly, the analysis device according to the embodiment of the present invention can improve the sensitivity for detecting diseases.

The conventional method determines that the count of exosomes 620 labeled by two beads 66 is two, although the number is actually one, which may result in a great variation between the counted value and the true value. For example, when the noise level Q is considered a point of reference, there is a risk in the conventional method that a specimen of which the concentration does not reach the point of reference may be subjected to imprecise analysis.

More particularly, with regard to ten thousand exosomes 620, when the number of exosomes 620 each bound to one bead 66 is eight thousand, and the number of exosomes 620 each bound to two beads 66 is two thousand, the count of exosomes 620 obtained by the method according to the embodiment of the present invention is ten thousand, while the count of exosomes 620 obtained by the conventional method results in twelve thousand. When the number of exosomes 620 each bound to one bead 66 is five thousand, and the number of exosomes 620 each bound to two beads 66 is five thousand, the count of exosomes 620 obtained by the method according to the embodiment of the present invention is ten thousand, while the count of exosomes 620 obtained by the conventional method results in fifteen thousand. When the number of exosomes 620 each bound to one bead 66 is two thousand, and the number of exosomes 620 each bound to two beads 66 is eight thousand, the count of exosomes 620 obtained by the method according to the embodiment of the present invention is ten thousand, while the count of exosomes 620 obtained by the conventional method results in eighteen thousand. Accordingly, the number of exosomes 620 each bound to one bead 66 and the number of exosomes 620 each bound to two beads 66 result in different ratios in each analysis.

The analysis device according to the embodiment of the present invention counts up the exosomes 620 in view of the pulse width of the detection signal depending on the arrangement of beads 66 when the beads 66 adjacent to each other are fixed onto the substrate 100. Therefore, the analysis device according to the embodiment of the present invention can count the exosomes 620 with high accuracy to improve the quantitative analysis of analytes even when irregular pulse waves are detected in the detection signal due to the arrangement of the beads 66.

Further, since the first reference value T2, the second reference value T4 and the third reference value T6 are determined in view of the jitter value of the detection signal, the analysis device according to the embodiment of the present invention can count the exosomes 620 with higher accuracy, so as to reduce the influence of jitter when classifying the pulse width Ta.

### <Other Embodiments>

While the present invention has been described above by reference to the embodiment, the present invention is not intended to be limited to the descriptions and drawings which form part of the disclosure. Various alternative embodiments, examples, and practical applications will be apparent to those skilled in the art from this disclosure.

For example, in the embodiment described above, the combination of the biomaterials as analytes and the specific biomaterials specifically binding to the analytes is not limited to the combination of the antigens 62 of the exosomes 620 and the antibodies 61 and antibodies 65 fixed to the beads 66. Examples of specifically-binding combinations include a combination of a ligand and an acceptor (such as enzymatic proteins, lectins, and hormones), and a combination of nucleic acids having base sequences complementing each other.

Alternatively, a well formed of, for example, silicone rubber may be provided on the surface of the substrate 100, and the reaction between the target antibodies 61, antigens 62 and beads 66 and the removal of materials not reacted by washing may be implemented within the well, so as to exclude the steps of, for example, spin washing and drying to simplify the process . Further, a plurality of wells may be provided in the same radius within the allowable area of the substrate 100, so as to measure a plurality of specimens simultaneously.

The present disclosure includes a program for executing, by a computer, the functions of a notifying device according to the embodiment described above. The program may be read out from a storage medium and input into the computer, or may be transmitted via an electrical communication circuit and input into the computer.

The scope of the present invention is defined only by the appropriate features according to the claims in view of the explanations made above.

### INDUSTRIAL APPLICABILITY

The present invention can provide an analysis device and an analysis method that can ensure improved quantitative analysis of analytes by varying the number to be added depending on a pulse width of a detection signal.

### REFERENCE SIGNS LIST

- 2: MOTOR
- 3: OPTICAL SCANNING UNIT
- 4: OPTICAL SYSTEM CONTROLLER
- 5: CONTROLLER
- 21: ROTATION CONTROLLER
- 31: LASER OSCILLATOR
- 32: COLLIMATOR LENS
- 33: BEAM SPLITTER
- 34: ACTUATOR
- 35: OBJECTIVE LENS
- 36: CONDENSING LENS
- 37: LIGHT DETECTOR
- 50: COUNTING UNIT
- 51: PULSE DETECTOR
- 52: STORAGE UNIT
- 61: ANTIBODY
- 62: ANTIGEN
- 63: SAMPLE SOLUTION
- 64: BUFFER SOLUTION
- 65: ANTIBODY
- 66: BEAD (PARTICLE)
- 100: SUBSTRATE
- 501: FIRST COUNTER
- 502: SECOND COUNTER
- 503: TARGET COUNTER
- 620: EXOSOME (ANALYTE)

## Claims

1. An analysis device comprising:
an optical scanning unit (3) configured to optically scan a surface of a substrate (100) to which analytes (620) in which particles (66) for labeling the analytes (620) are bound, are fixed, by using laser light having a wavelength longer than a diameter of the particle (66); and
a pulse detector (51) configured to detect a pulse wave and a pulse width (Ta) of the pulse wave included in a detection signal (D1, D2) obtained from the optical scanning unit (3) when the optical scanning unit (3) scans the substrate (100),
**characterized in that**
the analysis device further comprises a counting unit configured to determine that two particles (66) are bound to one analyte (620), when the pulse detector (51) detects two consecutive pulse waves each having a pulse width (Ta) less than a first reference value (T2), which is a width preliminary determined based on a first pulse width (T1) of one pulse wave among the two consecutive pulse waves, in a detection signal (D1, D2), and detects that a pulse interval (Tb) between the two pulse waves is less than a threshold (T6), and configured to count the number of analytes (620) as one when it is determined that two particles (66) are bound to one analyte (620).

2. The analysis device according to claim 1, wherein the counting unit determines that one particle (66) is bound to one analyte (620) when the pulse detector (51) detects a pulse wave having a pulse width (Ta) greater than or equal to the first reference value (T2), and less than a second reference value (T4), and counts the number of analytes (620) as one.

3. The analysis device according to claim 1 or 2, wherein, when the pulse detector (51) detects a first pulse wave having a pulse width (Ta) less than the first reference value (T2), detects a second pulse wave after the first pulse wave, and a pulse interval (Tb) between the first pulse wave and the second pulse wave is greater than or equal to the threshold (T6), the counting unit does not implement counting processing with regard to both the first pulse wave and the second pulse wave.

4. The analysis device according to claim 2, wherein, when the pulse detector (51) detects a first pulse wave having a pulse width (Ta) less than the first reference value (T2) and subsequently detects a pulse wave having the pulse width (Ta) greater than or equal to the first reference value (T2) and less than the second reference value (T4), the counting unit does not implement counting processing with regard to the first pulse wave.

5. The analysis device according to claim 2 or 4, wherein, when the pulse detector (51) detects a pulse wave having a pulse width (Ta) greater than or equal to the second reference value (T4), the counting unit does not implement counting processing with regard to the pulse wave having the pulse width (Ta) greater than or equal to the second reference value (T4).

6. The analysis device according to any one of claims 1 to 5, wherein the first reference value (T2) is a pulse width obtained by adding a predetermined value to the first pulse width (T1) of the pulse wave detected by the pulse detector (51), when the optical scanning unit (3) scans the two particles (66) which are bound to one analyte (620).

7. The analysis device according to any one of claims 2, 4 or 5, wherein the second reference value (T4) is a pulse width obtained by adding a predetermined value to a second pulse width of the pulse wave detected by the pulse detector (51), when the optical scanning unit (3) scans the one particles (66) which is bound to one analyte (620),

8. An analysis method comprising:
optically scanning a surface of a substrate (100) to which analytes (620) in which particles (66) for labeling the analytes (620) are bound, are fixed, by using laser light having a wavelength longer than a diameter of the particle (66); and
detecting a pulse wave and a pulse width (Ta) of the pulse wave included in a detection signal (D1, D2) obtained by scanning the substrate (100);
**characterized in that**
the analysis method further comprises:
determining that two particles (66) are bound to one analyte (620) when two consecutive pulse waves are detected each having a pulse width (Ta) less than a first reference value (T2), which is a width preliminary determined based on a first pulse width (T1) of one pulse wave among the two consecutive pulse waves, in the detection signal (D1, D2) and it is detected that a pulse interval (Tb) between the two pulse waves is less than a threshold (T6); and
counting the number of analytes (620) as one when it is determined that two particles (66) are bound to one analyte (620).

## Patentansprüche

1. Analysevorrichtung, die Folgendes aufweist:
eine optische Abtasteinheit (3), die konfiguriert ist, um optisch eine Oberfläche eines Substrates (100) zu scannen bzw. abzutasten, an dem Analyten (620) fixiert sind, an welche Partikel (66) zum Markieren der Analyten (620) gebunden sind, und zwar durch Verwendung von Laserlicht mit einer Wellenlänge, die länger ist als ein Durchmesser des Partikels (66); und
einen Impulsdetektor (51), der konfiguriert ist, um eine Impulswelle und eine Impulsbreite (Ta) der Impulswelle zu detektieren, die in einem Detektionssignal (D1, D2) enthalten ist, welches von der optischen Abtasteinheit (3) erhalten wurde, wenn die optische Abtasteinheit (3) das Substrat (100) abtastet,
**dadurch gekennzeichnet, dass**
die Analysevorrichtung weiter eine Zähleinheit aufweist, die konfiguriert ist, um zu bestimmen, dass zwei Partikel (66) an einen Analyten (620) gebunden sind, wenn der Impulsdetektor (51) zwei aufeinander folgende Impulswellen in einem Detektionssignal (D1, D2) detektiert, die jeweils eine Impulsbreite (Ta) von kleiner als einem ersten Referenzwert (T2) haben, was eine Breite ist, die zuvor basierend auf einer ersten Impulsbreite (T1) von einer Impulswelle aus den zwei aufeinander folgenden Impulswellen bestimmt wurde, und detektiert, dass ein Impulsintervall (Tb) zwischen den zwei Impulswellen kleiner als eine Schwelle (T6) ist und konfiguriert ist, um die Anzahl der Analyten (620) als eins zu zählen, wenn bestimmt wird,
dass zwei Partikel (66) an einen Analyten (620) gebunden sind.

2. Analysevorrichtung nach Anspruch 1, wobei die Zähleinheit bestimmt, dass ein Partikel (66) an einen Analyten (620) gebunden ist, wenn der Impulsdetektor (51) eine Impulswelle mit einer Impulsbreite (Ta) von größer als oder gleich dem ersten Referenzwert (T2) und kleiner als ein zweiter Referenzwert (T4) detektiert, und die Anzahl der Analyten (620) als eins zählt.

3. Analysevorrichtung nach Anspruch 1 oder 2, wobei, wenn der Impulsdetektor (51) eine erste Impulswelle mit einer Impulsbreite (Ta) von kleiner als dem ersten Referenzwert (T2) detektiert, eine zweite Impulswelle nach der ersten Impulswelle detektiert und ein Impulsintervall (Tb) zwischen der ersten Impulswelle und der zweiten Impulswelle größer als oder gleich der Schwelle (6) ist, die Zähleinheit nicht eine Zählungsverarbeitung bezüglich sowohl der ersten Impulswelle als auch der zweiten Impulswelle implementiert.

4. Analysevorrichtung nach Anspruch 2, wobei, wenn der Impulsdetektor (51) eine erste Impulswelle mit einer Impulsbreite (Ta) von kleiner als dem ersten Referenzwert (T2) detektiert, und darauf folgend eine Impulswelle mit einer Impulsbreite (Ta) von größer als oder gleich dem ersten Referenzwert (T2) und kleiner als dem zweiten Referenzwert (T4) detektiert, die Zähleinheit nicht die Zählverarbeitung bezüglich der ersten Impulswelle implementiert.

5. Analysevorrichtung nach Anspruch 2 oder 4, wobei, wenn der Impulsdetektor (51) eine Impulswelle mit einer Impulsbreite von größer als oder gleich dem zweiten Referenzwert (T4) detektiert, die Zähleinheit nicht die Zählverarbeitung bezüglich der Impulswelle mit der Impulsbreite (Ta) von größer als oder gleich dem zweiten Referenzwert (T4) implementiert.

6. Analysevorrichtung nach irgendeinem der Ansprüche 1 bis 5, wobei der erste Referenzwert (T2) eine Impulsbreite ist, die durch Addieren eines vorbestimmten Wertes zu der ersten Impulsbreite (T1) der Impulswelle erhalten wird, die durch den Impulsdetektor (51) detektiert wird, wenn die optische Abtasteinheit (3) die zwei Partikel (66) abtastet, die an einen Analyten (620) gebunden sind.

7. Analysevorrichtung nach irgendeinem der Ansprüche 2, 4 oder 5, wobei der zweite Referenzwert (T4) eine Impulsbreite ist, die erhalten wurde durch Addieren eines vorbestimmten Wertes zu einer zweiten Impulsbreite der Impulswelle, die durch den Impulsdetektor (51) detektiert wurde, wenn die optische Abtasteinheit (3) die einen Partikel (66) abtastet, die an einen Analyten (620) gebunden sind.

8. Analyseverfahren, welches Folgendes aufweist:
optisches Abtasten bzw. Scannen einer Oberfläche eines Substrates (100), an dem Analyten (620) fixiert sind, in denen Partikel (66) zum Markieren der Analyten (620) gebunden sind, und zwar durch Verwendung von Laserlicht mit einer Wellenlänge, die länger ist als ein Durchmesser des Partikels (66); und
Detektieren einer Impulswelle und einer Impulsbreite (Ta) der Impulswelle, die in einem Detektionssignal (D1, D2) enthalten ist, welches durch das Abtasten des Substrates (100) erhalten wurde;
**dadurch gekennzeichnet, dass**
das Analyseverfahren weiter Folgendes aufweist:
Bestimmen, dass zwei Partikel (66) an einen Analyten (620) gebunden sind, wenn zwei aufeinander folgende Impulswellen in dem Detektionssignal (D1, D2) detektiert werden, die jeweils eine Impulsbreite (Ta) von kleiner als einem ersten Referenzwert (T2) haben, was eine Breite ist, die zuvor basierend auf einer ersten Impulsbreite (T1) von einer Impulswelle von den zwei aufeinander folgenden Impulswellen bestimmt wurde, und detektiert wird, dass ein Impulsintervall (Tb) zwischen den zwei Impulswellen kleiner ist als eine Schwelle (T6); und
Zählen der Anzahl der Analyten (620) als eins, wenn bestimmt wird, dass zwei Partikel (66) an einen Analyten (620) gebunden sind.

## Revendications

1. Dispositif d'analyse comprenant :
une unité de balayage optique (3) configurée pour balayer optiquement une surface d'un substrat (100) sur laquelle des substances à analyser (620), auxquelles des particules (66) destinées à marquer les substances à analyser (620) sont liées, sont fixées, en utilisant une lumière laser ayant une longueur d'onde supérieure à un diamètre de la particule (66) ; et
un détecteur d'impulsions (51) configuré pour détecter une onde d'impulsion et une largeur d'impulsion (Ta) de l'onde d'impulsion comprise dans un signal de détection (D1, D2) obtenu à partir de l'unité de balayage optique (3) lorsque l'unité de balayage optique (3) balaie le substrat (100),
**caractérisé en ce que**
le dispositif d'analyse comprend en outre une unité de comptage configurée pour déterminer que deux particules (66) sont liées à une substance à analyser (620), lorsque le détecteur d'impulsions (51) détecte deux ondes d'impulsion consécutives ayant chacune une largeur d'impulsion (Ta) inférieure à une première valeur de référence (T2) qui est une largeur déterminée préliminairement sur la base d'une première largeur d'impulsion (T1) d'une onde d'impulsion parmi les deux ondes d'impulsion consécutives, dans un signal de détection (D1, D2), et détecte qu'un intervalle d'impulsion (Tb) entre les deux ondes d'impulsion est inférieure à un seuil (T6), et configuré pour compter le nombre de substances à analyser (620) comme étant un lorsqu'il est déterminé que deux particules (66) sont liées à une substance à analyser (620).

2. Dispositif d'analyse selon la revendication 1, dans lequel l'unité de comptage détermine qu'une particule (66) est liée à une substance à analyser (620) lorsque le détecteur d'impulsions (51) détecte une onde d'impulsion ayant une largeur d'impulsion (Ta) supérieure ou égale à la première valeur de référence (T2) et inférieure à une deuxième valeur de référence (T4), et compte le nombre de substances à analyser (620) comme étant un.

3. Dispositif d'analyse selon la revendication 1 ou 2, dans lequel, lorsque le détecteur d'impulsions (51) détecte une première onde d'impulsion ayant une largeur d'impulsion (Ta) inférieure à la première valeur de référence (T2), détecte une deuxième onde d'impulsion après la première onde d'impulsions et un intervalle d'impulsion (Tb) entre la première onde d'impulsion et la deuxième onde d'impulsion est supérieur ou égal au seuil (T6), l'unité de comptage ne met pas en œuvre de traitement de comptage s'agissant à la fois de la première onde d'impulsion et la deuxième onde d'impulsion.

4. Dispositif d'analyse selon la revendication 2, dans lequel, lorsque le détecteur d'impulsions (51) détecte une première onde d'impulsion ayant une largeur d'impulsion (Ta) inférieure à la première valeur de référence (T2) et détecte par la suite une onde d'impulsion ayant la largeur d'impulsion (Ta) supérieure ou égale à la deuxième valeur de référence (T4), l'unité de comptage ne met pas en œuvre de traitement de comptage s'agissant de la première onde d'impulsion.

5. Dispositif d'analyse selon la revendication 2 ou 4, dans lequel, lorsque le détecteur d'impulsions (51) détecte une onde d'impulsion ayant une largeur d'impulsion (Ta) supérieure ou égale à la deuxième valeur de référence (T4), l'unité de comptage ne met pas en œuvre de traitement de comptage s'agissant de l'onde d'impulsion ayant la largeur d'impulsion (Ta) supérieure ou égale à la deuxième valeur de référence (T4).

6. Dispositif d'analyse selon l'une quelconque des revendications 1 à 5, dans lequel la première valeur de référence (T2) est une largeur d'impulsion obtenue en ajoutant une valeur prédéterminée à la première largeur d'impulsion (T1) de l'onde d'impulsion détectée par le détecteur d'impulsions (51), lorsque l'unité de balayage optique (3) balaie les deux particules (66) qui sont liées à une substance à analyser (620).

7. Dispositif d'analyse selon l'une quelconque des revendications 2, 4 ou 5, dans lequel la deuxième valeur de référence (T4) est une largeur d'impulsion obtenue en ajoutant une valeur prédéterminée à une deuxième largeur d'impulsion de l'onde d'impulsion détectée par le détecteur d'impulsions (51), lorsque l'unité de balayage optique (3) balaie celle des particules (66) qui est liée à une substance à analyser (620).

8. Procédé d'analyse comprenant :
le balayage optique d'une surface d'un substrat (100) sur lequel des substances à analyser (620), auxquelles des particules (66) destinées à marquer les substances à analyser (620) sont liées, sont fixées, en utilisant une lumière laser ayant une longueur d'onde supérieure à un diamètre de la particule (66) ; et
la détection d'une onde d'impulsion et d'une largeur d'impulsion (Ta) de l'onde d'impulsion comprise dans un signal de détection (D1, D2) obtenu en balayant le substrat (100) ;
**caractérisé en ce que**
le procédé d'analyse comprend en outre :
la détermination que deux particules (66) sont liées à une substance à analyser (620) lorsque deux ondes d'impulsion consécutives sont détectées ayant chacune une largeur d'impulsion (Ta) inférieure à une première valeur de référence (T2), qui est une largeur déterminée préliminairement sur la base d'une première largeur d'impulsion (T1) d'une onde d'impulsion parmi les deux ondes d'impulsion consécutives, dans le signal de détection (D1, D2), et il est détecté qu'un intervalle d'impulsion (Tb) entre les deux ondes d'impulsion est inférieur à un seuil (T6) ; et
le comptage du nombre de substances à analyser (620) comme étant un lorsqu'il est déterminé que deux particules (66) sont liées à une substance à analyser (620).
